# EUROPEAN PATENT APPLICATION

(11) **EP 1 642 593 A1**
(43) Date of publication of application: **05.04.2006**
(21) Application number: 04735967.4
(22) Date of filing: 03.06.2004
(51) Int. Cl.: A61K 45/06, A61K 9/14, A61K 31/4439, A61K 31/366, A61K 45/00, A61P 3/10, A61P 9/10, A61P 29/00

(54) **SOLID PHARMACEUTICAL PREPARATION**

(30) Priority: 06.06.2003 JP 2003162241
(71) Applicant: Takeda Pharmaceutical Company Limited, Osaka 541-0045 (JP)
(72) Inventor: HAMAGUCHI, Naoru Takeda Pharmaceutical Comp. Ltd, Osaka (JP); KOYAMA, Hiroyoshi Takeda Pharmaceutical Comp. Ltd, Osaka (JP); MISAKI, Masafumi Takeda Pharmaceutical Comp. Ltd, Osaka (JP)
(74) Representative: Rickard, Timothy Mark Adrian
(86) International application number: PCT/JP2004/008076
(87) International publication number: WO 2004/108161

(57) **Abstract**

A solid preparation comprising an insulin sensitizer and an HMG-CoA reductase inhibitor without deteriorating the stabilities of these drugs, wherein said preparation comprises particles containing an insulin sensitizer and particles containing an HMG-CoA reductase inhibitor.

## Description

### Technical Field

The present invention relates to a solid preparation which comprises particles containing an insulin sensitizer and particles containing an HMG-CoA reductase inhibitor.

### Background Art

The combination use of an insulin sensitizer and an HMG-CoA reductase inhibitor is known to be useful for preventing and treating arteriosclerosis and xanthoma (see EP-A753298) as well as inflammatory diseases (see EP-A1254667) and others.

### Disclosure of Invention

The inventors of the present invention found that the compatibility between an insulin sensitizer and an HMG-CoA reductase inhibitor was not always good in preparing a solid preparation containing them.

The object of the present invention is to provide a solid preparation containing an insulin sensitizer and an HMG-CoA reductase inhibitor without deteriorating the stabilities of these drugs.

The inventors of the present invention had devoted themselves to attain the above-mentioned object and finally found that by using particles containing an insulin sensitizer and particles containing an HMG-CoA reductase inhibitor, a solid preparation could be formulated without deteriorating the stabilities of these drugs, resulting in completion of the present invention.

Namely, the present invention relates to,
(1) a solid preparation comprising particles containing an insulin sensitizer and particles containing an HMG-CoA reductase inhibitor;
(2) the solid preparation according to the above (1), wherein the insulin sensitizer is pioglitazone or a salt thereof;
(3) the solid preparation according to the above (1), wherein the insulin sensitizer is rosiglitazone or a salt thereof;
(4) the solid preparation according to the above (1), wherein the HMG-CoA reductase inhibitor is atorvastatin or a salt thereof;
(5) the solid preparation according to the above (1), wherein the HMG-CoA reductase inhibitor is pravastatin or a salt thereof;
(6) the solid preparation according to the above (1), wherein the HMG-CoA reductase inhibitor is simvastatin;
(7) the solid preparation according to the above (1), wherein the insulin sensitizer is pioglitazone or a salt thereof and the HMG-CoA reductase inhibitor is atorvastatin or a salt thereof;
(8) the solid preparation according to the above (1), wherein the insulin sensitizer is pioglitazone or a salt thereof and the HMG-CoA reductase inhibitor is pravastatin or a salt thereof;
(9) the solid preparation according to the above (1), wherein the insulin sensitizer is pioglitazone or a salt thereof and the HMG-CoA reductase inhibitor is simvastatin;
(10) the solid preparation according the above (1), which is a multi-layered tablet wherein the particles containing an insulin sensitizer and the particles containing an HMG-CoA reductase inhibitor are contained in separate layers; and others.

According to the present invention, a solid preparation containing an insulin sensitizer and an HMG-CoA reductase inhibitor wherein the stabilities of these drugs are not deteriorated (namely, degradation of or decrease in the activities of these drugs are prevented) can be obtained.

Further, according to the present invention, a solid preparation having excellent dissolution property of an insulin sensitizer and an HMG-CoA reductase inhibitor can be obtained.

Moreover, according to the present invention, by using an insulin sensitizer and an HMG-CoA reductase inhibitor as separate particles, any interaction can be avoided between these drugs or between these drugs and additives. Therefore, according to the present invention, various adverse effects (for example, degradation of or decrease in the activities of drugs; change in drug dissolution behavior from a preparation (for example; delay in drug dissolution)) which are caused by said interaction can be prevented.

Furthermore, according to the present invention, by using an insulin sensitizer and an HMG-CoA reductase inhibitor as separate particles, the dissolution behaviors of these drugs from a preparation can be individually controlled.

### Detailed Explanation of the Invention

An insulin sensitizer used in the present invention means any drug that restores the impaired function of an insulin receptor to the original state and thereby improves the insulin resistance, and specifically includes pioglitazone, rosiglitazone, reglixane (JTT-501), GI-262570, netoglitazone (MCC-555), YM-440, balaglitazone (DRF-2593), MB-13.1258, 5-(2,4-dioxothiazolidin-5-ylmethyl)-2-methoxy-N-[4-(trifluoromethyl)benzyl]benzamide (KRP-297), rivoglitazone (CS-011), FK-614, compounds described in WO99/58510 (e.g. (E)-4-[4-(5-methyl-2-phenyl-4-oxazolylmethoxy)benzyloxyimino]-4-phenylbutyric acid), tesaglitazar (AZ-242), ragaglitazar (NN-622), muraglitazar (BMS-298585), ONO-5816, LM-4156, MBX-102, LY-519818, MX-6054 and LY-510929.

The insulin sensitizer may be in the form of a salt. Such a salt may be a pharmacologically acceptable salt including salts with inorganic bases, salts with organic bases, salts with inorganic acids, salts with organic acids and salts with basic or acidic amino acids.

Preferred examples of the salts with inorganic bases include salts with alkali metals such as sodium and potassium; alkaline earth metals such as calcium and magnesium; aluminum, ammonium and the like.

Preferred examples of the salts with organic bases include salts with trimethylamine, triethylamine, pyridine, picoline, ethanolamine, diethanolamine, triethanolamine, dicyclohexylamine, N,N-dibenzylethylenediamine and the like.

Preferred examples of the salts with inorganic acids include salts with hydrochloric acid, hydrobromic acid, nitric acid, sulfuric acid, phosphoric acid and the like.

Preferred examples of the salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Preferred examples of the salts with basic amino acids include salts with arginine, lysine, ornithine and the like.

Preferred examples of the salts with acidic amino acids include salts with aspartic acid, glutamic acid and the like.

The insulin sensitizer may be anhydrous or hydrous and may be also labeled with an isotope (for example, ³H, ¹⁴C, ³⁵S, ¹²⁵I) or the like.

The insulin sensitizer used in the present invention may be a mixture of two or more kinds at an appropriate proportion.

The insulin sensitizer is preferably pioglitazone or a salt thereof (preferably, hydrochloride) or rosiglitazone or a salt thereof (preferably, maleate) and more preferably pioglitazone hydrochloride.

An HMG-CoA reductase inhibitor used in the present invention means any drug that inhibits 3-hydroxy-3-methylglutaryl-coenzyme A reductase (HMG-CoA reductase), which is an enzyme in rate-determining step of cholesterol biosynthesis, and specifically includes atorvastatin, pravastatin, cerivastatin, simvastatin, lovastatin, fluvastatin, itavastatin, rosuvastatin and pitavastatin.

The HMG-CoA reductase inhibitor may be in the form of a salt. Such a salt includes salts exemplified above for an insulin sensitizer.

Moreover, the HMG-CoA reductase inhibitor may be anhydrous or hydrous and may be also labeled with an isotope (for example, ³H, ¹⁴C, ³⁵S, ¹²⁵I) or the like.

The HMG-CoA reductase inhibitor used in the present invention may be a mixture of two or more kinds at an appropriate proportion.

The HMG-CoA reductase inhibitor is preferably atorvastatin or a salt thereof (preferably, calcium salt), pravastatin or a salt thereof (preferably, sodium salt), simvastatin, pitavastatin or a salt thereof (preferably, calcium salt), and more preferably simvastatin.

In the present invention, preferred combinations of an insulin sensitizer and an HMG-CoA reductase inhibitor include,
(1) a combination of pioglitazone or a salt thereof (preferably, hydrochloride) and atorvastatin or a salt thereof (preferably, calcium salt);
(2) a combination of pioglitazone or a salt thereof (preferably, hydrochloride) and pravastatin or a salt thereof (preferably, sodium salt);
(3) a combination of pioglitazone or a salt thereof (preferably, hydrochloride) and simvastatin;
(4) a combination of pioglitazone or a salt thereof (preferably, hydrochloride) and pitavastatin or a salt thereof (preferably, calcium salt);
(5) a combination of rosiglitazone or a salt thereof (preferably, maleate) and atorvastatin or a salt thereof (preferably, calcium salt);
(6) a combination of rosiglitazone or a salt thereof (preferably, maleate) and pravastatin or a salt thereof (preferably, sodium salt);
(7) a combination of rosiglitazone or a salt thereof (preferably, maleate) and simvastatin; and
(8) a combination of rosiglitazone or a salt thereof (preferably, maleate) and pitavastatin or a salt thereof (preferably, calcium salt).

A solid preparation of the present invention comprises particles containing an insulin sensitizer and particles containing an HMG-CoA reductase inhibitor (hereinafter, these particles may be simply referred to as the particles of the present invention).

The term "particles", when used herein, means particles with almost uniform shape and size, which can be obtained by granulating materials in powder, lump, solution or molten liquid form by a wet granulation method, a dry granulation method or a heated granulation method.

The particles of the present invention include powders, fine granules and granules, each of which preferably has particle size distribution prescribed in Japanese Pharmacopoeia 14th Edition.

Namely, according to Japanese Pharmacopoeia 14th Edition, when the particle size distribution test of preparations is performed, powders are defined preferably as "all the powders pass through a No. 18 (850 µm) sieve and not more than 5% of total powders remain on a No. 30 (500 µm) sieve", fine granules are defined preferably as "powders with not more than 10% of the total passing through a No. 200 (75 µm) sieve" among the above-mentioned powders, and granules are defined preferably as "all the granules pass through a No. 10 (1700 µm) sieve, not more than 5% of total granules remain on a No. 12 (1400 µm), and not more than 15% of total granules pass through a No. 42 (355 µm) sieve".

The average diameter of the particles of the present invention is usually 44 to 2000 µm and preferably 75 to 1000 µm.

Although the shape and size of the particles of the present invention may vary in process (for example, a compression process) of producing a solid preparation of the present invention, such particles that change in shape and size from the given original ones are also included in the particles of the present invention.

The repose angle of the particles of the present invention is preferably 55° or less and more preferably 50° or less, in view of filling into capsules, filling into pockets for divided powder, fluidity in tableting and filling into a mortar in tableting.

The solid preparation of the present invention and the particles of the present invention may contain additives conventionally used in the pharmaceutical technology field. Such additives include excipients, disintegrants, binders, lubricants, coloring agents, pH adjusters, surfactants, stabilizers, acidulants, flavors, fluidizing agents and sweetenings. A mixture of two or more these additives at an appropriate proportion may be used. The amounts used of additives are determined in accordance with quantities conventionally used in the pharmaceutical technology field.

Excipients include starches such as corn starch, potato starch, wheat starch, rice starch, partially pregelatinized (α) starch, pregelatinized (α) starch and porous starch; saccharides or sugar alcohols such as lactose, fructose, glucose, mannitol and sorbitol; anhydrous calcium phosphate, crystalline cellulose, precipitated calcium carbonate, and calcium silicate.

Disintegrants include carboxymethylcellulose, carboxymethylcellulose calcium, carboxymethylstarch sodium, croscarmellose sodium, crospovidone, low-substituted hydroxypropylcellulose and hydroxypropyl starch.

Binders include hydroxypropylcellulose, hydroxypropylmethylcellulose, polyvinylpyrrolidone and gum arabic powder.

Lubricants include magnesium stearate, calcium stearate, talc, sucrose fatty acid ester and sodium stearyl fumarate.

Coloring agents include food dyes such as food Yellow No. 5, food Red No. 2 and food Blue No. 2; food lake pigments and iron sesquioxide.

pH adjusters include citrate, phosphate, carbonate, tartrate, fumarate, acetate and amino acid salt.

Surfactants include polysorbate, polyoxyethylene hardened castor oil, polyoxyethylene (160) polyoxypropylene (30) glycol and sodium lauryl sulfate.

Stabilizers include sodium ascorbate, tocopherol, edetate tetrasodium, nicotinic acid amide, cyclodextrins; alkaline earth metal salts (for example, calcium carbonate, calcium hydroxide, magnesium carbonate, magnesium hydroxide, magnesium silicate, and magnesium aluminate), butylhydroxyanisole, ascorbic acid, and citric acid. The solid preparation and particles of the present invention preferably contain a stabilizer. The amount used of said stabilizer is, for example, 0.01 to 10 parts by weight, preferably 0.1 to 5 parts by weight per 100 parts by weight of an HMG-CoA reductase inhibitor. In the case where simvastatin is used as an HMG-CoA reductase inhibitor, in particular, it is preferable that at least one selected from butylhydroxyanisole, ascorbic acid and citric acid is used as a stabilizer.

Acidulants include ascorbic acid, citric acid, tartaric acid and malic acid.

Flavors include menthol, peppermint oil, lemon oil and vanillin.

Fluidizing agents include light anhydrous silicic acid and hydrous silicon dioxide. Light anhydrous silicic acid may contain hydrous silicon dioxide (SiO₂·nH₂O) (wherein n indicates an integer) as the main constituent and specifically includes Sylysia 320 (trade name; Fuji Silysia Chemical LTD) and AEROSIL 200 (trade name, Nippon Aerosil CO., LTD).

Sweetenings include Aspartame, Acesulfame K and saccharin sodium.

Additives include dissolution accelerators for active ingredients, namely, acids such as phosphoric acid, malonic acid, succinic acid, DL-malic acid, tartaric acid, maleic acid, fumaric acid and citric acid; and basic compounds such as sodium carbonate, sodium hydrogen carbonate, sodium citrate and sodium tartrate.

The particles of the present invention can be produced by granulating an insulin sensitizer or an HMG-CoA reductase inhibitor together with, if necessary, the above-mentioned additives, according to a conventional method in the pharmaceutical technology field. Granulation may be performed by wet granulation, dry granulation or heated granulation techniques and specifically, a high speed stirring granulator, a fluid bed granulator dryer, an extrusion granulator, a roller compactor, or the like can be used for granulation. After granulation, additional steps such as drying and sizing may be performed if necessary.

The content of an insulin sensitizer in the particles of the present invention is, for example, 0.01 to 100 parts by weight, preferably 0.1 to 90 parts by weight per 100 parts by weight of the particles of the present invention.

Specifically, when the insulin sensitizer is pioglitazone or a salt thereof (preferably, hydrochloride), the content of pioglitazone or a salt thereof is preferably 0.01 to 100 parts by weight, more preferably 1 to 90 parts by weight per 100 parts by weight of the particles of the present invention.

The content of an HMG-CoA reductase inhibitor in the particles of the present invention is, for example, 0.01 to 100 parts by weight, preferably 0.1 to 90 parts by weight per 100 parts by weight of the particles of the present invention.

Specifically, when the HMG-CoA reductase inhibitor is atorvastatin or a salt thereof (preferably calcium salt), pravastatin or a salt thereof (preferably sodium salt), simvastatin, or pitavastatin or a salt thereof (preferably calcium salt), the content of atorvastatin or a salt thereof, pravastatin or a salt thereof, simvastatin, or pitavastatin or a salt thereof is, for example, preferably 0.01 to 100 parts by weight, more preferably 1 to 90 parts by weight per 100 parts by weight of the particles of the present invention.

The dosage forms of the solid preparation of the present invention include oral preparations such as tablets (including sublingual tablets and intraorally disintegrating tablets), capsules (including soft capsules and microcapsules), powder, granules and troches; and parenteral preparations such as external preparations (for example, transdermal preparations and ointments), suppositories (for example, rectal suppositories and vaginal suppositories) and pellets. These preparations may be controlled-release preparations such as immediate-release preparations or sustained-release preparations (for example, sustained-release microcapsules).

The solid preparation of the present invention may be in round, caplet or oblong form.

The solid preparation of the present invention can be produced by formulating the particles of the present invention together with, if necessary, the above-mentioned additives according to a conventional method in the pharmaceutical technology field.

The formulation may be performed by combining granulation, mixing, filling into capsules, compression, coating and the like appropriately. The granulation is performed using a granulator such as a stirring granulator or a fluid bed granulator, the mixing is performed using a mixer such as a V-shape mixer or a tumbling mixer, and the compression is performed using, for example, a single-punch tableting machine or a rotary tableting machine and usually under a pressure of 0.3 to 35 kN/cm². The coating is performed using, for example, a film coating machine and a coating base may be, for example, a sugar-coating base, a water soluble film coating base, an enteric film coating base or sustained-release film coating base.

The sugar-coating base may be saccharose and one or more species selected from talc, precipitated calcium carbonate, gelatin, gum arabic, pullulan and carnauba wax may be used in combination.

The water soluble film coating base includes cellulose polymers such as hydroxypropylcellulose, hydroxypropylmethylcellulose, hydroxyethylcellulose and methylhydroxyethyl cellulose; synthesized polymers such as polyvinylacetal diethylaminoacetate, aminoalkylmethacrylate copolymer E [Eudragit E (trade name), Roehm Pharma] and polyvinylpyrrolidone; and polysaccharides such as pullulan.

The enteric film coating base includes cellulose polymers such as hydroxypropylmethylcellulose phthalate, hydroxypropylmethylcellulose acetate succinate, carboxymethylethylcellulose and cellulose acetate phthalate; acrylic acid polymers such as methacrylic acid copolymer L [Eudragit L (trade name), Roehm Pharma], methacrylic acid copolymer LD [Eudragit L-30D55 (trade name), Roehm Pharma] and methacrylic acid copolymer S [Eudragit S (trade name), Roehm Pharma]; and natural products such as shellac.

The sustained-release film coating base includes cellulose polymers such as ethyl cellulose; and acrylic acid polymers such as aminoalkylmethacrylate copolymer RS [Eudragit RS (trade name), Roehm Pharma] and ethyl acrylate/methyl methacrylate copolymer suspension [Eudragit NE (trade name), Roehm Pharma].

Two or more of the above-mentioned coating bases may be mixed at an appropriate proportion and then used. In a coating step, a light-blocking agent and/or a coloring agent such as titanium dioxide, talc, iron sesquioxide and yellow iron sesquioxide; a plasticizer such as polyethylene glycol, triethyl citrate, castor oil or polysorbates; or organic acid such as citric acid, tartaric acid, malic acid or ascorbic acid may be also used.

The solid preparation of the present invention may be printed with a mark or a letter for discrimination and may have a cleavage line for being divided.

Specific examples of the solid preparation of the present invention include,
(1) mixed powder obtained by mixing the particles of the present invention together with, if necessary, the above-mentioned additives;
(2) a capsule obtained by mixing the particles of the present invention together with, if necessary, the above-mentioned additives and then filling the mixture into a capsule (for example, a gelatin capsule);
(3) a molded product (for example, a tablet) obtained by mixing the particles of the present invention together with, if necessary, the above-mentioned additives and then compression molding the mixture;
(4) a molded product (for example, a dry-coated tablet) obtained by mixing one of the two kinds of particles of the present invention together with, if necessary, the above-mentioned additives and then compression molding the mixture to obtain a molded product (for example, a tablet), and mixing the other kind of particles together with, if necessary, the above-mentioned additives and then compressing the mixture around the above-mentioned molded product;
(5) a molded product (for example, a layered tablet (a multi-layered tablet)) obtained by mixing one of the two kinds of particles of the present invention together with, if necessary, the above-mentioned additives and then compression molding the mixture to obtain a molded product (for example, a tablet), and mixing the other kind of particles together with, if necessary, the above-mentioned additives and then compressing the mixture around the above-mentioned molded product in layered form; and
(6) a capsule obtained by filling any one of the molded products described in the above 3) to 5) (namely, a tablet, a dry-coated tablet and a layered tablet (a multi-layered tablet)) into a capsule (for example, a gelatin capsule).

In preparing the molded products described in the above 4) and 5) (namely, a dry-coated tablet and a layered tablet(a multi-layered tablet)), an intermediate layer of an inert additive (for example, an excipient) may be provided in order to prevent any direct contact between the drugs contained in the particles of the present invention.

Among the above-mentioned various molded products, the molded products described in the above 4) and 5) (namely, a dry-coated tablet and a layered tablet (a multi-layered tablet)) are preferable.

Namely, the solid preparation of the present invention is preferably a multi-layered tablet wherein particles containing an insulin sensitizer and particles containing an HMG-CoA reductase inhibitor are contained in separate layers. Said multi-layered tablet is preferably a two-layered tablet comprising (1) one layer containing particles of an insulin sensitizer and (2) the other layer containing particles of an HMG-CoA reductase inhibitor; or a three-layered tablet wherein an inert intermediate layer lies between these two layers.

As the solid preparation of the present invention, also preferred is the capsule described in the above 2). The particles of the present invention to be filled into said capsule are preferably granules.

The content of the particles of the present invention in the solid preparation of the present invention is, for example, 0.1 to 100 parts by weight, preferably 1 to 100 parts by weight per 100 parts by weight of the solid preparation of the present invention.

The content of an insulin sensitizer in the solid preparation of the present invention is, for example, 0.01 to 99 parts by weight, preferably 0.1 to 80 parts by weight per 100 parts by weight of the solid preparation of the present invention.

In the case where the insulin sensitizer is pioglitazone or a salt thereof (preferably, hydrochloride), the content of pioglitazone or a salt thereof in the solid preparation of the present invention is preferably 0.1 to 80 parts by weight, more preferably 1 to 50 parts by weight per 100 parts by weight of the solid preparation of the present invention.

The content of an HMG-CoA reductase inhibitor in the solid preparation of the present invention is, for example, 0.01 to 99 parts by weight, preferably 0.1 to 80 parts by weight per 100 parts by weight of the solid preparation of the present invention.

In the case where the HMG-CoA reductase inhibitor is atrovastatin or a salt thereof (preferably, calcium salt), pravastatin or a salt thereof (preferably, sodium salt), simvastatin, or pitavastatin or a salt thereof (preferably, calcium salt), the content of atrovastatin or a salt thereof, pravastatin or a salt thereof, simvastatin, or pitavastatin or a salt thereof in the solid preparation of the present invention is preferably 0.1 to 80 parts by weight, more preferably 1 to 50 parts by weight per 100 parts by weight of the solid preparation of the present invention.

The solid preparation of the present invention has less toxicity and can be orally or parenterally administered to mammals (for example, mice, rats, rabbits, cats, dogs, bovines, horses, monkeys, human being, and others) safely.

The solid preparation of the present invention is useful as a preventing and treating agent for, for example, glycometabolism disorder, lipidmetabolism disorder, diabetes (for example, type 1 diabetes, type 2 diabetes, gestational diabetes), hyperlipemia (for example, hypertriglyceridemia, (familial) hypercholesterolemia, hypo-high density lipoproteinemia, postprandial hyperlipemia), impaired glucose tolerance (IGT), diabetic complications [for example, neuropathy, nephropathy, retinopathy, cataract, macroangiopathy, osteopenia, diabetic hyperosmolar coma, infections (for example, respiratory tract infection, urinary tract infection, alimentary canal infection, dermal soft tissue infection, inferior limb infection), diabetic gangrene, xerostomia, hypacusis, cerebrovascular disorder, peripheral blood circulation disorder], obesity, osteoporosis, cachexia (for example, cancerous cachexia, tuberculous cachexia, diabetic cachexia, hemopathic cachexia, endocrinopathic cachexia, infectious cachexia, or AIDS-induced cachexia), fatty liver, hypertension, polycystic ovary syndrome, renal diseases (for example, diabetic nephropathy, glomerulonephritis, glomerulosclerosis, nephrotic syndrome, hypertensive nephrosclerosis, end-stage renal diseases), muscular dystrophy, myocardial infarction, angina pectoris, cerebrovascular disorder (for example, cerebral infarction, cerebral stroke), insulin resistant syndrome, syndrome X, dysmetabolic syndrome, hyperinsulinemia, hyperinsulinemia-induced sensory disorder, tumors (for example, leukemia, breast cancer, prostate cancer, skin cancer), irritable bowel syndrome, acute/chronic diarrhea, inflammatory diseases [for example, Alzheimer's disease, chronic rheumatoid arthritis, spondylitis deformans, arthritis deformans, lumbago, gout, postoperative or traumatic inflammation, swelling, neuralgia, pharyngitis, cystitis, hepatitis (including non-alcoholic fatty hepatitis), pneumonia, pancreatitis, inflammatory colonic disease, ulcerative colitis], visceral obesity syndrome, or arteriosclerosis (for example, atherosclerosis).

The solid preparation of the present invention is also useful for secondary prevention of the above-mentioned various diseases (for example, secondary prevention of cardiovascular events such as myocardial infarction) and inhibition of progression in these diseases (for example, inhibition of the progression from impaired glucose tolerance to diabetes, or inhibition of the progression to arteriosclerosis in diabetic patients).

A dose of the solid preparation of the present invention may be an effective amount based on an insulin sensitizer and an HMG-CoA reductase inhibitor contained in said solid preparation.

The effective amount of an insulin sensitizer is usually 0.01 to 500 mg/day, preferably 0.1 to 100 mg/day per adult (60 kg body weight).

In the case where the insulin sensitizer is pioglitazone hydrochloride, the effective amount of pioglitazone hydrochloride is usually 7.5 to 60 mg/day, preferably 15 to 45 mg/day per adult (60 kg body weight).

In the case where the insulin sensitizer is rosiglitazone maleate, the effective amount of rosiglitazone maleate is usually 1 to 12 mg/day, preferably 2 to 8 mg/day per adult (60 kg body weight).

The effective amount of an HMG-CoA reductase inhibitor is usually 0.01 to 500 mg/day per, preferably 1 to 100 mg/day per adult (60 kg body weight).

In the case where the HMG-CoA reductase inhibitor is atorvastatin calcium, the effective amount of atorvastatin calcium is usually 1 to 100 mg/day, preferably, 5 to 80 mg/day per adult (60 kg body weight).

In the case where the HMG-CoA reductase inhibitor is pravastatin sodium, the effective amount of pravastatin sodium is usually 1 to 100 mg/day, preferably 5 to 50 mg/day per adult (60 kg body weight).

In the case where the HMG-CoA reductase inhibitor is simvastatin, the effective amount of simvastatin is usually 1 to 160 mg/day, preferably 5 to 80 mg/day per adult (60 kg body weight) .

In the case where the HMG-CoA reductase inhibitor is pitavastatin calcium, the effective amount of pitavastatin calcium is usually 0.5 to 10 mg/day, preferably 1 to 4 mg/day per adult (60 kg body weight).

In the solid preparation of the present invention, the combination ratio between an insulin sensitizer and an HMG-CoA reductase inhibitor can be selected appropriately depending on a subject to be administered, disease to be treated, a combination of drugs and the like. For example, 0.01 to 100 parts by weight, preferably 0.1 to 10 parts by weight of an HMG-CoA reductase inhibitor may be usually used per 1 part by weight of an insulin sensitizer.

By using the solid preparation of the present invention, superior effects such as 1) enhanced actions of an insulin sensitizer and/or an HMG-CoA reductase inhibitor (for example, preventing and treating action of diabetes, hypercholesterolemia and the like), 2) reduced dosages of an insulin sensitizer and/or an HMG-CoA reductase inhibitor, 3) reduced adverse effects (for example, increase of body weight, rhabdomyolysis, myopathy, liver dysfunction, jaundice, hypersensitivity) of an insulin sensitizer and/or an HMG-CoA reductase inhibitor can be obtained as compared with single use of an insulin sensitizer or an HMG-CoA reductase inhibitor.

The solid preparation of the present invention may be used in combination with a concomitant drug which has no adverse effects on an insulin sensitizer or an HMG-CoA reductase inhibitor. Such a concomitant drug includes one or more selected from "diabetic treating agents (excluding insulin sensitizers)", "diabetic complication treating agents", "anti-obesity drugs", "hypotensive drugs", "antithrombotic drugs", "hyperlipemia treating agents (excluding HMG-CoA reductase inhibitors)" and "diuretics".

The "diabetic treating agents (excluding insulin sensitizers)" include insulin preparations (for example, animal-derived insulin preparations extracted from bovine or swine pancreas; human insulin preparations which are synthesized with genetic engineering using Escherichia coli or yeast; insulin zinc; protamine insulin zinc; insulin fragments or derivatives (for example, INS-1)), α,-glucosidase inhibitors (for example, voglibose, acarbose, miglitol, emiglitate), biguanide agents [for example, phenformin, metformin, buformin, or their salts (for example, hydrochloride, fumarate, succinate)], insulin secretagogues [sulfonylurea agents (for example, tolbutamide, glibenclamide, gliclazide, chlorpropamide, tolazamide, acetohexamide, glyclopyramide, glimepiride, glipizide, glybuzole), repaglinide, nateglinide, mitiglinide or its calcium salt hydrate, GLP-1], dipeptidylpeptidase IV inhibitors (for example, NVP-DPP-278, PT-100, NVP-DPP-728, LAF237, or the like), β3 agonists (for example, CL-316243, SR-58611-A, UL-TG-307, S-226552, AJ-9677, BMS-196085, AZ-40140), amylin agonists (for example, pramlintide), phosphotyrosine phosphatase inhibitors (for example, sodium vanadate), glyconeogenesis inhibitors (for example, glycogen phosphorylase inhibitors, glucose-6-phosphatase inhibitors, glucagon antagonists), and Sodium-glucose cotransporter (SGLUT) inhibitors (for example, T-1095) .

The "diabetic complication treating agents" include aldose reductase inhibitors (for example, tolrestat, epalrestat, zenarestat, zopolrestat, minalrestat, fidarestat (SNK-860), CT-112), neurotrophic factors (for example, NGF, NT-3, BDNF), neurotrophic factor production/secretion promotors [for example, neurotrophin production/secretion promotors described in WO01/14372, for example, 4-(4-chlorophenyl)-2-(2-methyl-1-imidazolyl)-5-(3-(2-methylphenoxy)propyl)oxazole)], PKC inhibitors (for example, LY-333531), AGE inhibitors (for example, ALT946, pimagedine, piratoxathin, N-phenacylthiazolium bromide (ALT766), EXO-226), active oxygen scavengers (for example, thioctic acid) and cerebral vasodilators (for example, tiapride, mexiletine).

The "anti-obesity drugs" include central anti-obesity drugs (for example, dexfenfluramine, fenfluramine, phentermine, sibutramine, amphepramone, dexanphetamine, mazindol, phenylpropanolamine, clobenzorex), pancreatic lipase inhibitors (for example, orlistat), β3 agonists (for example, CL-316243, SR-58611-A, UL-TG-307, SB-226552, AJ-9677, BMS-196085, AZ-40140), peptidic anorectics (for example, leptin, CNTF (ciliary neurotrophic factors)) and cholecystokinin agonists (for example, lintitript, FPL-15849).

The "hypotensive drugs" include angiotensin converting enzyme inhibitors (for example, captopril, enalapril, delapril), angiotensin II antagonists (for example, candesartan, cilexetil, losartan, eprosartan, valsartan, telmisartan, irbesartan, tasosartan), calcium antagonists (for example, manidipine, nifedipine, nicardipine, amlodipine, efonidipine), potassium channel openers (for example, levcromakalim, L-27152, AL 0671, NIP-121), and clonidine.

The "antithrombotic drugs" include heparin (for example, heparin sodium, heparin calcium, dalteparin sodium), warfarin (for example, warfarin potassium), anti-thrombin agents (for example, aragatroban), thrombolytic agents (for example, urokinase, tisokinase, alteplase, nateplase, monteplase, pamiteplase), platelet aggregation inhibitors (for example, ticlopidine hydrochloride), cilostazol, ethyl icosapentate, beraprost sodium and sarpogrelate hydrochloride.

The "hyperlipemia treating agents (excluding HMG-CoA reductase inhibitors)" include fibrate compounds (for example, benzafibrate, beclobrate, binifibrate, ciprofibrate, clinofibrate, clofibrate, clofibric acid, etofibrate, fenofibrate, gemfibrozil, nicofibrate, pirifibrate, ronifibrate, simfibrate, theofibrate), squalene synthase inhibitors (for example, the compounds described in WO97/10224, for example, 1-[[(3R, 5S)-1-(3-acetoxy-2,2-dimethylpropyl)-7-chloro-5-(2,3-dimethoxyphenyl)-2-oxo-1,2,3,5-tetrahydro-4,1-benzoxazepine-3-yl]acetyl]piperidine-4-acetic acid), ACAT inhibitors (for example, Avasimibe, Eflucimibe), anion-exchange resins (for example, cholestyramine), probucol and nicotinic acid drugs (for example, nicomol, niceritrol, ethyl icosapentate, phytosterol (for example, soysterol), γ-oryzanol).

The "diuretics" include xanthine derivatives (for example, sodium salicylate theobromine, calcium salicylate theobromine), thiazide agents (for example, ethiazide, cyclopenthiazide, trichlormethiazide, hydrochlorothiazide, hydroflumethiazide, benzylhydrochlorothiazide, penflutizide, polythiazide and methyclothiazide), anti-aldosterone drugs (for example, spironolactone, triamterene), carbonic anhydrase inhibitors (for example, acetazolamide), chlorobenzenesulfonamide drugs (for example, chlorthalidone, mefruside, indapamide), azosemide, isosorbide, etacrynic acid, piretanide, bumetanide and furosemide.

The timing of administration of the solid preparation of the present invention and a concomitant drug is not limited and they may be administered simultaneously or at staggered times. Alternatively, a single dosage form containing the solid preparation of the present invention and a concomitant drug may be administered to a subject.

A dose of a concomitant drug can be selected appropriately based on the clinical dose. The combination ratio between the solid preparation of the present invention and a concomitant drug can be selected appropriately depending on a subject to be administered, an administration route, disease to be treated, symptoms and a combination of drugs. In the case where a subject to be administered is a human, 0.01 to 100 parts by weight of a concomitant drug may be used per 1 part by weight of the solid preparation.

Thus, by using a concomitant drug, superior effects such as 1) enhanced actions of the solid preparation of the present invention and a concomitant drug (synergistic action of the drugs), 2) reduced dosages of the solid preparation of the present invention or a concomitant drug (reduction in dosage compared with those when they are administered individually) and 3) reduced adverse effects of the solid preparation of the present invention and a concomitant drug can be obtained.

Hereinafter, the present invention will be explained in detail with reference to Reference Examples, Examples and Experimental Examples which are not intended to limit the present invention.

In the following Reference Examples and Examples, products that meet the Japanese Pharmacopoeia 14th Edition were used as various additives such as magnesium stearate. The symbol % shown in the Reference Examples and Examples indicates % by weight, unless otherwise specified.

### Reference Example 1 (Production of mixed powder containing atorvastatin calcium)

Atorvastatin calcium (43.4 g) that is previously ground with an atomizer grinding machine (Fuji Paudal CO., LTD, Model K-II-1, screen size 2.0 mmø), calcium carbonate (75.2 g) (NITTO FUNKA KOGYO KK), crystalline cellulose (136.6 g) (trade name: PH101, Asahi KASEI Corporation), lactose (54.4 g) (Maigret) and croscarmellose sodium (FMC) (10.2 g) are put in a fluid bed granulator (POWREX Corporation, LAB―1). Thereto a solution of polysorbate 80 (1.36 g) (trade name: RHEODOL TW-0120, Kao Corporation) and hydroxypropylcellulose (6.84 g) (NISSO) in water (116 mL) is sprayed and the mixture is granulated and dried to obtain granules. To the resultant granules (311.6 g) are added croscarmellose sodium (9.73 g) and magnesium stearate (1.63 g) (TAIHEI CHEMICAL INDUSTRIAL CO., LTD) to obtain mixed powder.

### Reference Example 2 (Production of mixed powder containing atorvastatin calcium)

Atorvastatin calcium (43.4 g) that is previously ground with an atomizer grinding machine (Fuji Paudal CO., LTD, Model K-II-1, screen size 2.0 mmo) and calcium carbonate (75.2 g), crystalline cellulose (136.6 g), lactose (54.4 g) and croscarmellose sodium (10.2 g) are put in a fluid bed granulator (POWREX Corporation, LAB-1). Thereto a solution of polysorbate 80 (1.36 g), sodium ascorbate (0.07 g) and hydroxypropylcellulose (6.84 g) in water (116 mL) is sprayed and the mixture is granulated and dried to obtain granules. To the resultant granules (311.6 g) are added croscarmellose sodium (9.73 g) and magnesium stearate (1.63 g) to obtain mixed powder.

### Reference Example 3 (Production of mixed powder containing pioglitazone hydrochloride)

Pioglitazone hydrochloride (132.2 g), lactose (305.4 g) and carmellose calcium (14.4 g) (Gotoku Chemical Company LTD.) are put in a fluid bed granulator (PAUREX Corporation, LAB-1). Thereto a solution of hydroxypropylcellulose (12.0 g) in water (188 mL) is sprayed and the mixture is granulated and dried to obtain granules. To the resultant granules (440.8 g) are added carmellose calcium (13.7 g) and magnesium stearate (1.52 g) to obtain mixed powder.

### Reference Example 4 (Production of mixed powder containing simvastatin)

Simvastatin (50.1 g) containing 0.3% of butylhydroxyanisole, lactose (296.25 g), crystalline cellulose (100 g) and citric acid (6.25 g) were put in a fluid bed granulator (PAUREX COPORATION LAB-1). Thereto a solution of hydroxypropylcellulose (15 g) in water (250 mL) was sprayed and the mixture was granulated and dried to obtain 450 g of granules. The above described process was repeated to obtain another 453 g of granules. To the resultant granules (903 g) were added crospovidone (48.24 g) (ISP) and magnesium stearate (14.48 g) to obtain mixed powder.

### Reference Example 5 (Production of mixed powder containing simvastatin)

Simvastatin (60.12 g) containing 0.3% of butylhydroxyanisole, lactose (409.5 g), pregelatinized starch (30 g) (NIPPON STARCH CHEMICAL CO.,LTD.) and citric acid (7.5 g) were put in a fluid bed granulator (PAUREX COPORATION, LAB-1). Thereto a dispersion prepared by dispersing pregelatinized starch (30 g) in 33% (v/v) ethanol (125 mL) and then adding water (375 mL) was sprayed and the mixture was granulated and dried to obtain 518 g of granules. The above described process was repeated to obtain another 528 g of granules. To the resultant granules (1046 g) were added crystalline cellulose (58.42 g), crospovidone (46.74 g) and magnesium stearate (17.53 g) to obtain mixed powder.

### Reference Example 6 (Production of mixed powder containing pioglitazone hydrochloride)

Pioglitazone hydrochloride (20.5 kg), lactose (44.31 kg) and croscarmellose calcium (4.464 kg) were put in a fluid bed granulator (PAUREX COPORATION, FD-WSG-60). Thereto 2.232 kg of polyvinylpyrrolidone K30 (BASF) was sprayed using a solution of polyvinylpyrrolidone K30 (3.732 kg) in water (33.59 L), and the mixture was granulated and dried to obtain granules. To the resultant granules (1000 g) were added croscarmellose sodium (36.42 g) and magnesium stearate (4.16 g) to obtain mixed powder.

### Example 1 (Production of a capsule)

The mixed powder of Reference Example 1 (85 g) and the mixed powder of Reference Example 3 (120 g) are mixed in a plastic bag. The resultant mixture (205 mg) is filled into a No. 0 gelatin capsule to obtain a capsule containing 10 mg of atorvastatin and 30 mg of pioglitazone.

### Example 2 (Production of a capsule)

Into a No. 0 gelatin capsule, the mixed powder of Reference Example 1 (85 mg) and then the mixed powder of Reference Example 3 (120 mg) are filled to obtain a capsule containing 10 mg of atorvastatin and 30 mg of pioglitazone.

### Example 3 (Production of divided powder)

The mixed powder of Reference Example 1 (85 g) and the mixed powder of Reference Example 3 (120 g) are mixed in a plastic bag. The resultant mixture is filled into laminated polyethylene bags in an amount of 205 mg per a bag to obtain divided powder containing 10 mg of atorvastatin and 30 mg of pioglitazone per a bag.

### Example 4 (Production of a layered tablet)

The mixed powder of Reference Example 1 (85 mg) is compressed using a tableting machine (Kikusui Seisakusho LTD., Tableting Machine for Layered Tablets) under a pressure of 0.5 kN/cm² and further, the mixed powder of Reference Example 3 (120 mg) is compressed under a pressure of 5 kN/cm² to obtain a layered tablet. The resultant tablets (205 g) are put in a coating machine (Freund, HCT-20) and coated with a film suspension of hydroxypropylmethylcellulose (62.2 g) (trade name: TC-5, Shin-Etsu Chemical Co., LTD), titanium dioxide (12.5 g) (Ishihara Sangyo Co., LTD.), polyethylene glycol 6000 (8.32 g) (Sanyo Kasei CO., LTD.), yellow iron sesquioxide (0.12 g) (Ansted) and water (960 g) so as to attain 6 mg of coating per a tablet. Thus a film coated tablet containing 10 mg of atorvastatin and 30 mg of pioglitazone is obtained.

### Example 5 (Production of a layered tablet)

The mixed powder of Reference Example 2 (170 mg) is compressed using a tableting machine (Kikusui Seisakusho LTD., Tableting Machine for Layered Tablets) under a pressure of 0.5 kN/cm² and further, the mixed powder of Reference Example 3 (120 mg) is compressed under a pressure of 5 kN/cm² to obtain a layered tablet. The resultant tablets (205 g) are put in a coating machine (Freund, HCT-20) and coated with a film suspension of hydroxypropylmethylcellulose (62.2 g), titanium dioxide (12.5 g), polyethylene glycol 6000 (8.32 g), yellow iron sesquioxide (0.12 g) and water (960 g) so as to attain 9 mg of coating per a tablet. Thus a film coated tablet containing 20 mg of atorvastatin and 30 mg of pioglitazone is obtained.

### Example 6 (Production of a dry-coated tablet)

The mixed powder of Reference Example 1 (85 mg) is compressed using a tableting machine (Kikusui Seisakusho LTD., Tableting Machine for Dry coated Tablets) under a pressure of 5 kN/cm² to obtain a tablet. The resultant tablet as a core and the mixed powder of Reference Example 3 (180 mg) as an outer layer are compressed into a tablet. Thus a dry-coated tablet containing 10 mg of atorvastatin and 45 mg of pioglitazone is obtained.

### Example 7 (Production of a capsule)

Into a No. 0 gelatin capsule, the mixed powder of Reference Example 5 (200 mg) and then the mixed powder of Reference Example 6 (120 mg) were filled to obtain a capsule containing 20 mg of simvastatin and 30 mg of pioglitazone.

### Example 8 (Production of a layered tablet)

The mixed powder of Reference Example 4 (400 mg) was compressed using a tableting machine (Kikusui Seisakusho LTD., Tableting Machine for Layered Tablets) under a pressure of 0.5 kN/cm² and further, the mixed powder of Reference Example 6 (180 mg) was compressed under a pressure of 12 kN/cm² to obtain a layered tablet containing 40 mg of simvastatin and 45 mg of pioglitazone.

### Example 9 (Production of a layered tablet)

The mixed powder of Reference Example 5 (400 mg) was compressed using a tableting machine (Kikusui Seisakusho LTD., Tableting Machine for Layered Tablets) under a pressure of 0.5 kN/cm² and further, the mixed powder of Reference Example 6 (180 mg) was compressed under a pressure of 13 kN/cm² to obtain a layered tablet containing 40 mg of simvastatin and 45 mg of pioglitazone.

### Example 10 (Production of a film coated tablet)

The layered tablets (140 g) obtained in Example 9 was put in a coating machine (Freund, HCT-20) and coated with a film suspension of hydroxypropylmethylcellulose (149.2 g), titanium dioxide (20 g), polyethylene glycol 6000 (30 g), iron sesquioxide (0.8 g) and water (2000 g) so as to attain 17.4 mg of coating per a tablet. Thus a film coated tablet containing 40 mg of simvastatin and 45 mg of pioglitazone was obtained.

### Example 11

The mixed powder of Reference Example 4 (200 mg) is compressed using a tableting machine (Kikusui Seisakusho LTD., Tableting Machine for Layered Tablets) under a pressure of 0.5 kN/cm² and further, the mixed powder of Reference Example 6 (120 mg) is compressed under a pressure of 12 kN/cm² to obtain a layered tablet containing 20 mg of simvastatin and 30 mg of pioglitazone.

### Example 12

The mixed powder of Reference Example 4 (400 mg) is compressed using a tableting machine (Kikusui Seisakusho LTD., Tableting Machine for Layered Tablets) under a pressure of 0.5 kN/cm² and further, the mixed powder of Reference Example 6 (120 mg) is compressed under a pressure of 12 kN/cm² to obtain a layered tablet containing 40 mg of simvastatin and 30 mg of pioglitazone.

### Example 13

The mixed powder of Reference Example 5 (200 mg) is compressed using a tableting machine (Kikusui Seisakusho LTD., Tableting Machine for Layered Tablets) under a pressure of 0.5 kN/cm² and further, the mixed powder of Reference Example 6 (120 mg) is compressed under a pressure of 13 kN/cm² to obtain a layered tablet containing 20 mg of simvastatin and 30 mg of pioglitazone.

### Example 14

The mixed powder of Reference Example 5 (400 mg) is compressed using a tableting machine (Kikusui Seisakusho LTD., Tableting Machine for Layered Tablets) under a pressure of 0.5 kN/cm² and further, the mixed powder of Reference Example 6 (120 mg) is compressed under a pressure of 13 kN/cm² to obtain a layered tablet containing 40 mg of simvastatin and 30 mg of pioglitazone.

### Experimental Example

The dissolution of simvastatin and pioglitazone from the layered tablet obtained in Example 9 was tested by a paddle method (50 rev). Ten mM phosphate buffer (37°C, pH 7.0) containing 0.5% sodium dodecyl sulfate and 0.3M potassium chloride-hydrochloric acid buffer (37°C, pH 2.0) were used as dissolution media for Simvastatin and for Pioglitazone, respectively. The result is shown in Table 1.

**Table 1 Dissolution rate of Drug (%)**

| Preparation | Drug | Dissolution rate (%) | | |
|---|---|---|---|---|
| | | 10 min. after | 20 min. after | 30 min. after |
| Example 9 | Simvastatin | 98 | 98 | 99 |
| | Pioglitazone | 85 | 93 | 96 |

As shown in Table 1, the solid preparation of the present invention has good dissolution property of an insulin sensitizer (pioglitazone) and an HMG-CoA reductase inhibitor (simvastatin).

### Industrial Applicability

According to the present invention, a solid preparation containing an insulin sensitizer and an HMG-CoA reductase inhibitor, wherein the stabilities of these drugs are not deteriorated can be obtained.

In addition, according to the present invention, a solid preparation having excellent dissolution property of an insulin sensitizer and an HMG-CoA reductase inhibitor can be obtained.

## Claims

1. A solid preparation comprising particles containing an insulin sensitizer and particles containing an HMG-CoA reductase inhibitor.

2. The solid preparation according to claim 1, wherein the insulin sensitizer is pioglitazone or a salt thereof.

3. The solid preparation according to claim 1, wherein the insulin sensitizer is rosiglitazone or a salt thereof.

4. The solid preparation according to claim 1, wherein the HMG-CoA reductase inhibitor is atorvastatin or a salt thereof.

5. The solid preparation according to claim 1, wherein the HMG-CoA reductase inhibitor is pravastatin or a salt thereof.

6. The solid preparation according to claim 1, wherein the HMG-CoA reductase inhibitor is simvastatin.

7. The solid preparation according to clam 1, wherein the insulin sensitizer is pioglitazone or a salt thereof and the HMG-CoA reductase inhibitor is atorvastatin or a salt thereof.

8. The solid preparation according to claim 1, wherein the insulin sensitizer is pioglitazone or a salt thereof and the HMG-CoA reductase inhibitor is pravastatin or a salt thereof.

9. The solid preparation according to claim 1, wherein the insulin sensitizer is pioglitazone or a salt thereof and the HMG-CoA reductase inhibitor is simvastatin.

10. The solid preparation according to claim 1, which is a multi-layered tablet wherein the particles containing an insulin sensitizer and the particles containing an HMG-CoA reductase inhibitor are contained in separate layers.
